(19) Europäisches Patentamt
European Patent Office
Office européen des brevets

(11) Veröffentlichungsnummer : **0 155 610 B2**

(12) **NEUE EUROPÄISCHE PATENTSCHRIFT**

(45) Veröffentlichungstag der neuen Patentschrift : **08.01.92 Patentblatt 92/02**

(51) Int. Cl.$^5$ : **C07D 209/88**

(21) Anmeldenummer : **85102698.9**

(22) Anmeldetag : **09.03.85**

# (54) Verfahren zur Herstellung von 2-Hydroxy-(9H)-carbazol.

(30) Priorität : **21.03.84 DE 3410232**

(43) Veröffentlichungstag der Anmeldung : **25.09.85 Patentblatt 85/39**

(45) Bekanntmachung des Hinweises auf die Patenterteilung : **04.03.87 Patentblatt 87/10**

(45) Bekanntmachung des Hinweises auf die Entscheidung über den Einspruch : **08.01.92 Patentblatt 92/02**

(84) Benannte Vertragsstaaten : **BE CH DE FR GB IT LI NL**

(56) Entgegenhaltungen :
**JOURNAL OF THE CHEMICAL SOCIETY, 1955, London J.A. CUMMINS et al.: "Hydroxycarbazoles and Tetrahydrohydroxycarbazoles. Part III."**

(56) Entgegenhaltungen :
**CHEMISCHE BERICHTE, 92. Jahrgang, nr. 7, 1959, Verlag Chemie GmbH., Weinheim/Bergstrasse H.-J. TEUBER et al. "Tetrahydrocarbazole und Tetrahydrocarbazolchinone, III" Seiten 2385-2399**
**Chemical Reviews vol. 78, 1978 . pp 317 ff**
**Journal Am. Chem. Soc.vol. 71, 1949 p.2964**
**Journal of organic chem., vol. 24, 1959 p 478**
**Organic syntheses coll.vol.IV p.536**
**Journal American chem. soc. vol. 91 1969,p 4535**
**Bulletin chem.soc. of Japan vol.11, 1936, p.218**

(73) Patentinhaber : **HOECHST AKTIENGESELLSCHAFT**
**Postfach 80 03 20**
**W-6230 Frankfurt am Main 80 (DE)**

(72) Erfinder : **Schweikert, Otto Ernst, Dr.**
**Freiherr-vom-Stein-Strasse 37**
**W-6233 Kelkheim (Taunus) (DE)**
Erfinder : **Reimann, Walter, Dr.**
**Thüringer Weg 33**
**W-6238 Hofheim am Taunus (DE)**
Erfinder : **Wykypiel, Werner, Dr.**
**Egerstrasse 7**
**W-6054 Rodgau (DE)**
Erfinder : **Mack, Karl Ernst, Dr.**
**Klingenbachstrasse 43**
**W-6200 Wiesbaden (DE)**

EP 0 155 610 B2

## Beschreibung

Die Erfindung betrifft ein Verfahren zur Herstellung von 2-Hydroxy-(9H)-carbazol (HC) durch katalytische Dehydrierung von 1,2,3,4-Tetrahydro-7-hydroxy-(9H)-carbazol (THC).

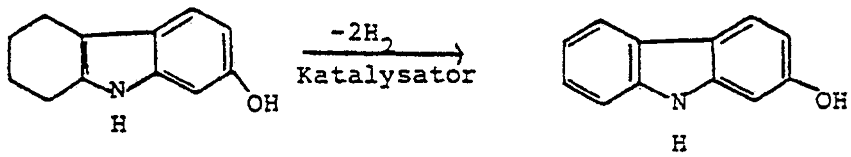


THC HC

HC ist ein wichtiges Zwischenprodukt zur Herstellung von Farbstoffen; dabei wird i. allg. aus HC zunächst Azylsäure (2-Hydroxy-(9H)-carbazol-1-carbonsäure) hergestellt.

Verfahren zur Herstellung von HC sind bereits bekannt. Nach US-PS 4137 238 wird HC durch Cyclisierung von 2-Chlor-3'-hydroxydiphenylamin mit Basen hergestellt. Nachteilig hierbei ist das Arbeiten in verdünnten Lösungen (geringe Raum-Zeit-Ausbeuten). Außerdem fallen durch die erforderliche Neutralisierung des Reaktionsproduktes salzhaltige Abwässer an.

Die Herstelllung von HC durch katalytische Dehydrierung von THC wird beschrieben in J. Chem. Soc. 1955, Seite 3475. THC wird mit Pd/C gemischt und in Abwesenheit eines Lösungsmittels in einer $CO_2$-Atmosphäre auf 270 °C erhizt. Auch dieses Verfahren hat jedoch den Nachteil, daß das Reaktionsprodukt mit Natronlauge behandelt werden muß, so daß zunächst das Na-Salz des HC entsteht, aus dem durch Behandlung mit Säure HC gewonnen werden muß. Angaben über die Ausbeuten werden von den Autoren nicht gemacht.

Ziel der vorliegenden Erfindung ist es, ein umweltfreundliches Verfahren zur Herstellung von HC durch Dehydrierung des nach Literatur (Chem. Ber. 92, 2385 (1959)) zugänglichen THC zur Verfügung zu stellen.

Das Verfahren zur Herstellung von 2-Hydroxy-(9H)-carbazol durch katalytische Dehydrierung von 1,2,3,4-Tetrahydro-7-hydroxy-(9H)-carbazol ist dadurch gekennzeichnet, daß die Reaktion in einem Alkohol oder einem Etheralkohol oder deren Gemischen durchgeführt wird.

Es ist ein Vorteil des neuen Verfahrens, daß HC ohne Zusatz weiterer Hilfsstoffe auf einfache Weise aus der vom Katalysator befreiten Reaktionslösung isoliert werden kann. Dies geschieht im allgemeinen dadurch, daß das Lösungsmittel vollständig oder teilweise abgedampft wird und HC auskristallisiert. Das abgedampfte und kondensierte Lösungsmittel wird erneut verwendet.

Die beim erfindungsgemäßen Verfahren eingesetzten Alkohole und Etheralkohole, sowie deren Gemische weisen im allgemeinen unterschiedliche Lösevermögen für das Ausgangsprodukt THC und das Endprodukt HC auf, so daß HC nach Abkühlen der Reaktionslösung in hoher Reinheit isoliert werden kann. Vor dem Abkühlen wird ggf. das Lösungsmittel teilweise abgedampft, bzw. werden bei Verwendung eines Lösungsmittelgemisches dessen leichter siedende und das THC gut lösende Komponenten abgedampft. Gleichzeitig erlaubt das Auskristallisieren von HC aus der Reaktionslösung den Einsatz von THC geringerer Reinheit als Ausgangsmaterial. Geeignete Alkohole und Etheralkohole sind z.B.: Amylalkohol, Cyclohexanol, 2-Ethylhexanol, Octanol, Decanol, Methylglykol, n-Butylglykol und t-Butylglykol.

Bevorzugt werden: 2-Ethylhexanol, Octanol und n-Butylglykol. Der erfolgreiche Einsatz von Alkoholen und Etheralkoholen ist insofern überraschend, als aus der Literatur bekannt ist, daß diese Verbindungen bei erhöhter Temperatur in Gegenwart von Hydrier- bzw. Dehydrierkatalysatoren zur raschen Abspaltung von CO und $H_2$ neigen.

Die Reaktionstemperaturen liegen im Bereich von 120-300°C, vorzugsweise bei 140-220°C, insbesondere bei 170-190°C. Im allgemeinen wird die Reaktion bei der Siedetemperatur des gewählten Lösungsmittels durchgeführt. In diesem Fall wird die maximale Reaktionstemperatur durch den Siedepunkt des Lösungsmittels vorgegeben, der durch Anwendung von Druck auch erhöht werden kann. Falls gewünscht, kann der Siedepunkt des Lösungsmittels auch durch Anwendung von Unterdruck verringert werden.

Als Katalysatoren eignen sich alle üblichen Hydrier- bzw. Dehydrierkatalysatoren wie Palladium, Ruthenium, Rhodium, Iridium, Platin und Nickel in fein verteilter Form oder auf Trägern. Auch Gemische dieser Metalle sind für die Reaktion geeignet.

Als Träger können beispielsweise verwendet werden : Kohle, $SiO_2$, $Al_2O_3$, Aluminiumsilikate, Spinelle, Chromoxid-Aluminiumoxide und Zeolithe. Die Konzentration des Metalls auf dem Träger beträgt dabei im allgemeinen 0.1-15 Gew.-%. Der Katalysator wird nach beendeter Reaktion abfiltriert und kann erneut verwendet werden.

Das erfindungsgemäße Verfahren kann kontinuierlich oder diskontinuierlich durchgeführt werden.

Die folgenden Beispiele sollen das erfindungsgemäße Verfahren erläutern.

Vergleichs beispiel 1

187 g THC wurden in 1.5 l Dimethyldiglykol gelöst. Nach Zugabe von 50 g Pd/Kohle (10 Gew.-% Pd) als Katalysator wurde die Apparatur mit Stickstoff gespült und anschließend 3 h lang unter Rühren auf 165 °C erhitzt. Innerhalb dieser Zeit wurden 42.5 Nl Wasserstoff entwickelt. Nach Abkühlen des Reaktionsgemisches wurde der Katalysator abfiltriert und das Filtrat zur Trockne eingedampft. Der Rückstand wurde gemörsert und im Vakuum (70 °C, 50 mbar) 4 h lang getrocknet. Man erhielt 180.4 g rohes HC, welches 96.4 Gew.-% HC, 2.3 Gew.-% Dimethyldiglykol und noch 1.3 Gew.-% nicht umgesetztes THC enthielt. Dies entspricht einer Ausbeute an reinem HC von 95 %.

Beispiel 2

187 g THC (Reinheitsgrad : 70 %) wurden in 1.5 l 2-Ethylhexanol unter Erwärmen gelöst, mit 100 g Pd-/Kohle (5 Gew.-% Pd) versetzt und mit Stickstoff überlagert. Das Gemish wurde unter Rühren 3 h lang auf 185 °C erhitzt, wobei 41 Nl Wasserstoff freigesetzt wurden. Der Katalysator wurde heiß abfiltriert und mit 0.5 l heißem 2-Ethylhexanol ausgewaschen. Das Filtrat wurde auf ein Volumen von 750 ml eingedampft und anschließend auf Raumtemperatur abgekühlt. Das auskristallisierte HC wurde abgesaugt und im Vakuum (70 °C, 50 mbar) getrocknet. Man erhielt 111 g HC (Reinheitsgrad : 98 %), das bei 276 °C schmolz. Ausbeute : 85 % an reinem HC.

Beispiel 3

374 g THC (Reinheitsgrad: 84%) wurden in einem Gemisch aus 1.5 l 2-Ethylhexanol und 0.5 l n-Butylglykol gelöst, mit 100 g Pd/Kohle (5 Gew.-% Pd) versetzt und mit Stickstoff überlagert. Das Gemisch wurde 4 h lang auf 180°C erhitzt, wobei 71 Nl Wasserstoff entwichen. Der Katalysator wurde heiß abfiltriert und mit 0.5 l heißem Lösungsmittelgemisch ausgewaschen. Das Filtrat wurde über eine 50 cm-Füllkörperkolonne auf ein Volumen von 1.5 l eingedampft, wobei hauptsächlich das niedrig siedende n-Butylglykol abdestilliert wurde. Nach Abkühlen auf Raumtemperatur wurde das auskristallisierte HC abgesaugt und im Vakuum (70 °C, 50 mbar) getrocknet. Man erhielt 287 g HC (Reinheitsgrad : 98.5 %). Ausbeute : 92 % an reinem HC.

## Patentansprüche

1. Verfahren zur Herstellung von 2-Hydroxy-(9H)-carbazol durch katalytische Dehydrierung von 1,2,3,4 - Tetrahydro-7-hydroxy-(9H)-carbazol, dadurch gekennzeichnet, daß die Reaktion in einem Alkohol oder einem Etheralkohol oder deren Gemischen durchgeführt wird.

2. Verfahren nach Anspruch 1, dadurch gekennzeichnet, daß die Reaktion in Amylalkohol, Cyclohexanol, 2-Ethylhexanol, Octanol, Decanol, Methylglykol, n-Butylglykol, t-Butylglykol oder deren Gemischen durchgeführt wird.

3. Verfahren nach Anspruch 1, dadurch gekennzeichnet, daß die Reaktion in 2-Ethylhexanol, Octanol, n-Butylglykol oder deren Gemischen durchgeführt wird.

## Claims

1. A process for the preparation of 2-hydroxy-(9H)-carbazole by catalytic dehydrogenation of 1,2,3,4-tetrahydro-7-hydroxy-(9H)-carbazole, which comprises carrying out the reaction in an alcohol or an ether alcohol or mixtures thereof.

2. The process as claimed in claim 1, wherein the reaction is carried out in amyl alcohol, cyclohexanol, 2-ethylhexanol, octanol, decanol, methylglycol, n-butylglycol, t-butylglycol or mixtures thereof.

3. The process as claimed in claim 1, wherein the reaction is carried out in 2-ethylhexanol, octanol, n-butylglycol or mixtures thereof.

**Revendications**

1. Procédé de préparation de l'hydroxy-2 (9H)-carbazole par déshydrogénation catalytique du tétrahydro-1, 2,3,4 hydroxy-7 (9H)-carbazole, procédé caractérisé en ce qu'on effectue la réaction dans un alcool ou un éther-alcool ou leurs mélanges.

2. Procédé selon la revendication 1 caractérisé en ce qu'on effectue la réaction dans du pentanol, cyclohexanol, de l'étyl-2 hexanol, de l'octanol, du décanol, du metyl-glycol, du n-butyl-glycol, du tert-butyl-glycol ou des mélanges de ceux-ci.

3. Procédé selon la revendication 1 caractérisé en ce qu'on effectue la réaction dans de l'éthyl-2 hexanol, de l'octanol, du n-butyl-glycol ou des mélanges de ceux-ci.